# EUROPEAN PATENT SPECIFICATION

(11) **EP 2 281 205 B1**
(45) Date of publication and mention of the grant of the patent: **20.07.2011**
(21) Application number: 09746236.0
(22) Date of filing: 11.05.2009
(51) Int. Cl.: G01P 13/00, G01P 15/08, G08B 21/02

(54) **FALL DETECTION SYSTEM**
FALLDETEKTIONSSYSTEM
SYSTÈME DE DÉTECTION DE CHUTE

(30) Priority: 13.05.2008 CN 200810099512
(43) Date of publication of application: 09.02.2011
(73) Proprietor: Koninklijke Philips Electronics N.V., 5621 BA Eindhoven (NL)
(72) Inventor: CHEN, Ningjiang, 5656 AE Eindhoven (NL); KIM, Sheng, 5656 AE Eindhoven (NL); VAN DEN DUNGEN, Wilhelmus, A., M., A., M., 5656 AE Eindhoven (NL); BAGGEN, Constant, P., M., J., 5656 AE Eindhoven (NL); DOORNBOS, Richard, M., P., 5656 AE Eindhoven (NL)
(74) Representative: Van Velzen, Maaike Mathilde
(86) International application number: PCT/IB2009/051924
(87) International publication number: WO 2009/138941

(56) References cited:
- EP-A- 1 188 625
- WO-A-02/061704
- US-A- 4 829 285
- US-A- 6 094 141
- US-A1- 2007 088 521
- CHEN ET AL: "Wearable Sensors for Reliable Fall Detection" ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE, 1 January 2005 (2005-01-01), pages 3551-3554, XP031001295 ISBN: 978-0-7803-8741-6

## Description

### TECHNICAL FIELD OF THE INVENTION

The invention relates to a fall detection system, and in particular to a method and apparatus for improving the battery life of a fall detection system.

### BACKGROUND TO THE INVENTION

There is a need for activity detector systems that can detect if a person has fallen badly and needs medical assistance. Although many elderly people have personal help buttons (PHBs) that they can push if they need urgent assistance, if they have had a bad fall, they may not be able to reach or push the PHB, which means there can be a significant delay from the fall taking place and assistance reaching the person.

Therefore, fall detection systems have been proposed that are worn by a user, and that determine whether the user has fallen based on measurements of the motion of the user by one or more sensors.

A fall is an event that can be characterized (usually) by at least four particular features. These are, in a rough chronological order, (i) a quick downward acceleration of the body; (ii) a decrease in altitude; (iii) an impact when the body hits the ground; and (iv) a change in orientation of the body, from being upright to lying down.

In designing a wearable fall detection system for an elderly user, it is important to make sure that the system is easily maintained, i.e. by reducing the requirement to recharge or replace the battery. Thus, it is desirable to prolong the battery life of the fall detection system for as long as possible.

The proposed fall detection systems have a micro-controller or processor for processing the measurements from the sensors. In many fall detection systems, the processor obtains measurements from multiple sensors and computes several features in order to detect whether or not a fall event has occurred. A key for successful power management is to make a good decision on when and how to process the measurements from the sensors.

Fall detection systems are known that use the measurements obtained from one sensor to "wake up" the rest of the device. One such system is that provided by Health Watch (httr)://www.health-watch.com/falldetector.html) which wakes from a standby mode if a first sensor detects a rapid change in the orientation of the user, and which can then monitor for an impact.

However, as this fall detection system uses a sensor that detects a change in orientation to wake up a sensor that detects an impact, and as it has been found that the impact can happen before the change in orientation, this system may not detect all types of fall.

WO 02/061704 A (ILIFE SOLUTIONS INC [US]) 8 August 2002 (2002-08-08) discloses an apparatus and system for detecting a fall, and also for shutting down selected parts of the system where no fall event is being detected.

CHEN ET AL: "Wearable Sensors for Reliable Fall Detection" ENGINEERING IN MEDICINE AND BIOLOGY SOCIETY, 2005. IEEE-EMBS 2005. 27TH ANNUAL INTERNATIONAL CONFERENCE OF THE SHANGHAI, CHINA 01-04 SEPT. 2005, PISCATAWAY, NJ, USA,IEEE, 1 January 2005 (2005-01-01), pages 3551-3554, XP031001295 ISBN: 978-0-7803-8741-6 discloses a system for 'waking up' sensors and associated processor circuitry used to monitor post fall conditions in response to a heavy impact e.g. a fall.

US-A-6 094 141 (TSAI CHING-TIEN [TW]) 25 July 2000 (2000-07-25) discloses a system employing a passive sensor switch which detects movement in order to activate electrical illumination devices. In the event of there being no movement these illuminations are deactivated, thus conserving power.

An additional consideration is that many fall detection algorithms require historic information about the motion of the user in order to arrive at a correct result when determining if a fall has taken place, and, given the short timeframe within which a fall can occur, it is necessary for the system to respond quickly to an event detected by one or more of the sensors. Therefore, continuously waking up the system to collect and store the historical information, as well as maintaining the sampling rate of the sensors at a suitable level to avoid events falling between samples uses a large amount of energy.

Therefore, there is a need for a method and apparatus for improving the battery life of a fall detection system.

### SUMMARY OF THE INVENTION

According to a first aspect of the invention, there is provided a fall detection system comprising a passive vibration sensor; one or more other sensors for detecting respective characteristics of a fall; and a processor for analyzing measurements from the one or more other sensors to determine if a fall has occurred; wherein the system is configured to selectively provide power to the one or more other sensors and/or processor in response to the passive vibration sensor detecting motion of a user of the fall detection system.

Therefore, by using the passive vibration sensor to activate other components in the fall detection system when motion is detected, the power consumption of the system is significantly reduced (and hence the battery lifetime is increased) during periods when there is no or little motion.

In one embodiment, the system further comprises a passive tilt sensor; and the system is configured to provide power to the one or more other sensors in response to the passive vibration sensor detecting motion of a user, and to provide power to the processor in the event that the measurements from the tilt sensor indicate a characteristic of a fall.

In an alternative embodiment, the system is configured to provide power to the one or more other sensors in response to the passive vibration sensor detecting motion of a

user, and to provide power to the processor such that the processor is in a low power mode for analyzing measurements from the other sensors to identify characteristics of a fall.

Preferably, the processor is configured to switch to a high power mode in the event that the measurements from one of the other sensors indicate a characteristic of a fall.

Preferably, the system is configured to stop power being supplied to the one or more other sensors and processor in the event that the passive vibration sensor detects that the motion has ceased.

In an alternative embodiment, the processor comprises a simple low power processor and a main processor, and the system is configured to provide power to the simple processor in response to the passive vibration sensor detecting motion.

Preferably, the simple processor is configured to increase a sampling rate of the one or more other sensors in response to the passive vibration sensor detecting motion.

Preferably, the simple processor is configured to analyze the measurements from the one or more other sensors to identify a characteristic of a fall.

Preferably, the simple processor is configured to activate the main processor in the event that the simple processor detects a characteristic of a fall.

Preferably, the simple processor is configured to reduce the sampling rate of the one or more other sensors in response to the passive vibration sensor detecting that the motion has ceased.

Preferably, the one or more other sensors comprises an accelerometer and/or a barometer.

According to a second aspect of the invention, there is provided a method of operating a fall detection system comprising a passive vibration sensor, one or more other sensors for detecting respective characteristics of a fall, and a processor for analyzing measurements from the one or more other sensors to determine if a fall has occurred; the method comprising the step of selectively providing power to the one or more other sensors and/or processor in response to the passive vibration sensor detecting motion of a user of the fall detection system.

### BRIEF DESCRIPTION OF THE DRAWINGS

The invention will now be described, by way of example only, with reference to the following drawings, in which:
Fig. 1 is a representation of the six states that occur before, during and after a fall;
Fig. 2 is a block diagram of a fall detection system in accordance with a first embodiment of the invention;
Figs. 3 and 4 are flow charts illustrating alternative methods of operating the fall detection system shown in Fig. 2;
Fig. 5 is a block diagram of a fall detection system in accordance with a second embodiment of the invention.

### DETAILED DESCRIPTION OF THE PREFERRED EMBODIMENTS

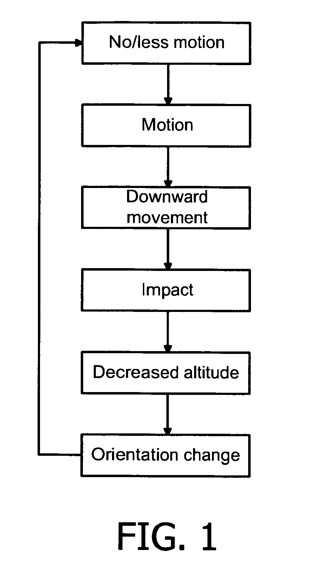

In accordance with an aspect of the invention, a state machine framework can be used to determine a power-efficient operation of a fall detection system. Figure 1 shows the states that occur before, during and after a fall, in the order in which computations are carried out by a processor. Thus, as described above, initially there will be no or little motion, for example when a user of the fall detection system is sitting or lying down. If the user stands up and starts walking, there will be some motion. If the user starts to fall, there will be a downward movement, followed by an impact as the user hits the ground, a decrease in altitude and change in orientation as the user's body moves from being upright to lying down. If the fall has been severe enough that assistance is needed, then there will again be no or little motion.

It will be noted that the state machine framework indicates that the computation for the decrease in altitude occurs after the computation for the detection of an impact, as it is necessary to use data from before and after the impact to determine the decrease in altitude.

As different sensors are used to detect the different movements that occur during a fall, for example an accelerometer can detect the downward movement and the impact, a barometer can detect the decrease in altitude, and a tilt sensor can be used to detect the change in orientation, the state machine framework can be used to determine an order in which the various sensors can be powered on, in order for them to take the measurements needed to determine whether a fall has taken place. In other words, maximizing the time that power-hungry sensors are deactivated helps to reduce the power consumption (and hence increase the battery life) of the fall detection system.

In particular, it can be seen from the state machine representation that certain conditions can be specified and which must be met in order for the sensor or sensors and a processor that analyses the measurements from the sensors to be powered on. Until the conditions are met, the sensors or processor can remain in a standby mode that consumes little or no power.

In all embodiments of the invention, there are four general principles that can reduce the power consumption of the fall detection system. The first principle is the use of as many passive sensors (i.e. those that do not require electrical energy to operate) as possible in place of active sensors that do require power to operate. The second principle is the use of the passive sensors to detect activity, and for these sensors to "wake up" the other sensors and/or the processor in the system. The third principle is for the most power efficient active sensors to be woken up first (i.e. a barometer is generally more power efficient than an accelerometer, so should be woken up first. The fourth principle is that the sampling rate of the sensors can be varied depending on the current state in order to minimize the power consumption when little or no motion is occurring.

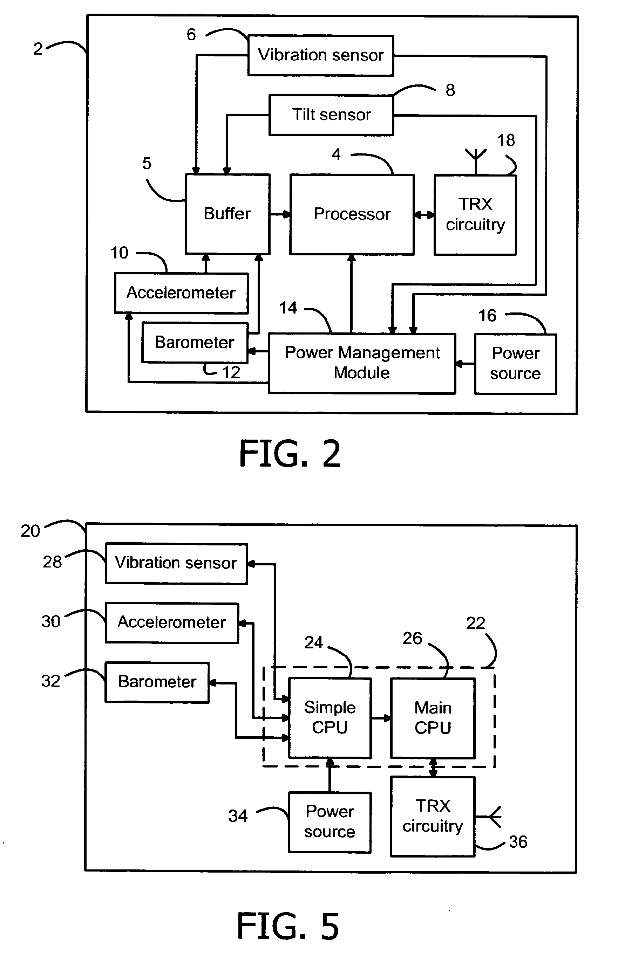

A first embodiment of a fall detection system 2 is shown in Figure 2. The fall detection system 2 comprises a processor 4 connected to a buffer 5, that is itself connected to a plurality of sensors 6, 8, 10 and 12. At least one of the sensors is a passive sensor, and in this embodiment, the passive sensor is a vibration sensor 6 that detects motion of the user of the fall detection system 2 and can be a simple "switch-type" sensor. A further passive sensor is the tilt sensor 8, which detects the tilt of the fall detection system 2, and in particular the change in orientation of the user that can occur in a fall. In some embodiments of the invention, the tilt sensor 8 can be omitted from the fall detection system 2.

The remaining sensors 10, 12 in this fall detection system are active sensors, which means that they require electrical power to operate. Sensor 10 is an accelerometer that measures the accelerations experienced by the user (such as a downward movement and an impact), and sensor 12 is a barometer that measures a change in altitude of the user. As with the tilt sensor 8, the barometer 12 can be omitted from the system 2 in certain implementations, in which case measurements from the accelerometer 10 can be used to estimate the change in altitude of the user.

Each of the plurality of sensors 6, 8, 10 and 12 are connected to the buffer 5, so that measurements by the sensors are stored therein for use in a fall detection algorithm executed by the processor 4.

The fall detection system 2 also comprises a power management module 14 that is connected to the processor 4, accelerometer 10 and barometer 12 for selectively providing power from a power source 16 to these components. The power management module 14 is also connected to the vibration sensor 6 and tilt sensor 8, and operates in response to the measurements from these sensors 6, 8. The power management module 14 preferably comprises a simple combination of switches and logic circuitry that consumes little power from the power source 16.

Finally, the fall detection system 2 comprises transceiver circuitry 18 that is connected to the processor 4 and which is used to transmit an alarm signal if a fall is detected.

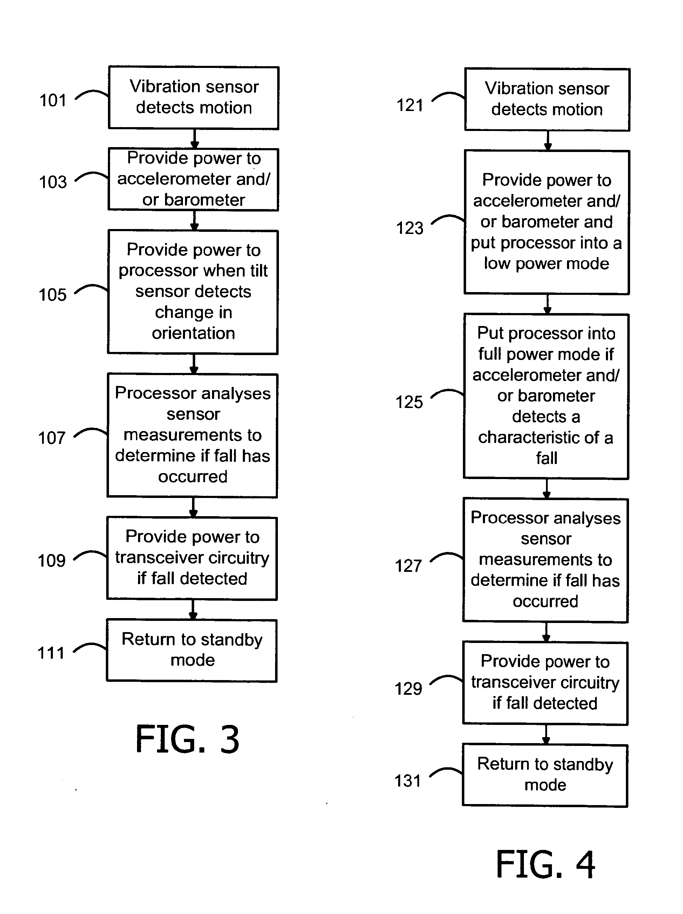

A flow chart illustrating the operation of the fall detection system 2 is shown in Figure 3. Initially, before any movements characteristic of a fall have occurred or been detected, the power management module 14 puts the system 2 into a "standby" state when there is little or no motion. In this state, the power management module 14 powers off the processor 4, accelerometer 10 and barometer 12, so that the system 2 is using little or no electrical energy from the power source 16.

In step 101, the passive vibration sensor 6 detects motion of the user, and this is indicated to the power management module 14. Then, as shown in step 103, the power management module 14 selectively provides power to the accelerometer 10 and barometer 12 so that these sensors start to collect and store measurements in the buffer 5.

If the passive tilt sensor 8 then detects a change in orientation of the user that is above a threshold (for example if a fall has occurred), the power management module 14 additionally provides power to the processor 4 so that the processor 4 can start to execute the fall detection algorithm using the measurements made by the sensors 6, 8, 10 and 12 stored in the buffer 5 (steps 105 and 107).

If a fall is detected by the processor 4, power can be provided to the transceiver circuitry 18 so that an alarm signal can be sent out (step 109). The power can preferably be selectively provided to the transceiver circuitry 18 by the processor 4, but can alternatively be provided to the transceiver circuitry 18 by the power management module 14.

Once the alarm signal has been transmitted, the power management module 14 returns the fall detection system 2 to the standby mode in which the processor 4, accelerometer 10 and barometer 12 are powered down (step 131).

A flow chart illustrating an alternative operation of the fall detection system 2 is shown in Figure 4. In this system 2, the processor 4 has two different modes of operation, a low-power mode and a full-power mode, in addition to a powered-down mode. In this illustration, the tilt sensor 8 is not present in the system 2. As above, before any movements characteristic of a fall have occurred or been detected, the power management module 14 puts the system 2 into a "standby" state when there is little or no motion. In this state, the power management module 14 powers off the processor 4, accelerometer 10 and barometer 12, so that the system 2 is using little or no electrical energy from the power source 16.

In step 121, the passive vibration sensor 6 detects motion of the user, and this is indicated to the power management module 14. Then, as shown in step 123, the power management module 14 selectively provides power to the accelerometer 10 and barometer 12 so that these sensors start to collect and store measurements in the buffer 5. In addition, the power management module 14 provides power to the processor 4 so that it operates in the low-power mode.

If the processor 4, operating in the low-power mode, determines from the measurements by the accelerometer 10 or barometer 12 that a downward motion, impact, decrease in altitude or any other characteristic of a fall has occurred, the processor 4 switches into the full-power mode so that it can start to execute the fall detection algorithm using the measurements made by the sensors 6, 10 and 12 stored in the buffer 5 (steps 125 and 127).

If a fall is detected by the processor 4 when it is operating in the full power mode, power can be provided to the transceiver circuitry 18 so that an alarm signal can be sent out (step 129). The power can preferably be selectively provided to the transceiver circuitry 18 by the processor 4, but can alternatively be provided to the transceiver circuitry 18 by the power management module 14.

Once the alarm signal has been transmitted, the power management module 14 returns the fall detection system 2 to the standby mode in which the processor 4, accelerometer 10 and barometer 12 are powered down (step 131).

Figure 5 shows a fall detection system in accordance with a second embodiment of the invention. In this embodiment, it is necessary for the sensors to periodically take measurements, so that the processor has historic measurements available for when it executes the fall detection algorithm.

The system 20 according to this second embodiment comprises a processing unit 22, which includes a simple processor 24 and a main processor 26. The system 20 comprises a plurality of sensors 28, 30 and 32, namely a passive vibration sensor, an accelerometer and a barometer respectively. It will be appreciated that this system 20 could further include a tilt sensor. Each of the sensors 28, 30 and 32 is connected to the simple processor 24.

The power source 34 for the system 20 is also connected to the simple processor 24, and the simple processor 24 selectively provides this power to the main processor 26, accelerometer 30 and barometer 32. Transceiver circuitry 36 is connected to the main processor 26. It will be appreciated from the description of this embodiment that the simple processor 24 has effectively replaced the power management module 14 and buffer 5 of the first embodiment shown in Figure 2.

As described above, in this embodiment, the sensors 30 and 32 are periodically required to take measurements to ensure that historic information is available. When the vibration sensor 28 detects no or little motion, the simple processor 24 controls the other sensors 30 and 32 so that they have a low sampling rate, i.e. they wake up every t₁ seconds to take measurements, which are stored in a memory of the simple processor 24.

However, if the vibration sensor 28 detects motion (or motion above a threshold), the vibration sensor 28 can wake up the simple processor 24, so that it increases the sampling rate of the sensors 30 and 32 so that they wake up every tₕ seconds (where tₕ > t₁) to take measurements. As the simple processor 24 can take this action at any time (i.e. not just when the sensors 30 and 32 are already active), it is possible for the sampling rate to be increased as soon as an event (i.e. motion) occurs. If this event happens to be the start of a fall, the first measurement can be made by the sensor 30 and 32 within around 2ms of the event occurring or starting.

In addition, when motion occurs, the simple processor 24 performs basic calculations on the measurements (such as threshold tests) to try to identify characteristics of a fall. If the simple processor 24 determines that a fall might be occurring (for example if there has been an acceleration that is above a threshold, or if the altitude of the user has changed by more than a predetermined amount), then it provides power to the main processor 26 so that the full fall detection algorithm can be executed on the measurements.

If the main processor 26 determines that a fall has not taken place, it is switched off, and the simple processor 24 continues to monitor the measurements made by the sensors 30 and 32 for further potential indicators of a fall.

If the main processor 26 determines that a fall has taken place, it provides power to the transceiver circuitry 36 and an alarm signal is transmitted. The main processor 26 then powers down.

If the motion detected by the vibration sensor 28 ceases (whether or not the simple processor 24 has woken the main processor 26), the simple processor 24 returns the sensors 30 and 32 to the lower sampling rate and enters a sleep or standby mode.

If the simple processor 24 determines that a fall event is unlikely to be occurring (for example if the threshold tests are not satisfied), but the vibration sensor 28 indicates that motion is occurring, then the simple processor 24 continues to monitor the measurements for an indicator of a fall, but does not wake the main processor 26.

When sampling is not taking place, all possible components of the system 20, including the simple processor 24 as far as possible (for example excluding a timer indicating when the sensors 30 and 32 should make the next measurement), should be put into a low power or standby mode, so that power consumption of the system 20 is significantly reduced.

Thus, when the vibration sensor 28 indicates that there is no or little motion, the sampling rate is reduced in order to conserve energy, while providing for the possibility for the sampling rate to be increased as soon as it becomes necessary.

Therefore, there is provided a method and apparatus for improving the battery life of a fall detection system by using a passive vibration sensor to activate other components of the system when motion is detected.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The more fact that certain measures are recited in mutually different dependent claims does not indicate that a combination of these measured cannot be used to advantage. Any reference signs in the claims should not be construed as limiting the scope. A computer program may be stored/distributed on a suitable medium, such as an optical storage medium or a solid-state medium supplied together with or as part of other hardware, but may also be distributed in other forms, such as via the Internet or other wired or wireless telecommunication systems.

## Claims

1. A fall detection system (2) for detecting a fall of a user, comprising:
a passive vibration sensor (6) requiring no electrical energy to operate;
one or more other sensors (10, 12) for detecting a movement characteristic of a fall; and
a processor (4) for analyzing measurements from the one or more other sensors to determine if a fall has occurred;
wherein the system is configured to selectively provide power to the one or more other sensors and/or processor in response to the passive vibration sensor detecting motion of the user of the fall detection system.

2. A fall detection system (2) as claimed in claim 1, the system further comprising a passive tilt sensor (8); wherein the system is configured to provide power to the one or more other sensors (10, 12) in response to the passive vibration sensor (6) detecting motion of a user, and to provide power to the processor in the event that the measurements from the tilt sensor indicate a characteristic of a fall.

3. A fall detection system (2) as claimed in claim 1, wherein the system is configured to provide power to the one or more other sensors (10, 12) in response to the passive vibration sensor (6) detecting motion of a user, and to provide power to the processor (4) such that the processor is in a low power mode for analyzing measurements from the other sensors to identify characteristics of a fall.

4. A fall detection system (2) as claimed in claim 3, wherein the processor (4) is configured to switch to a high power mode in the event that the measurements from one of the other sensors (10, 12) indicate a characteristic of a fall.

5. A fall detection system (2) as claimed in any preceding claim, wherein the system is configured to stop power being supplied to the one or more other sensors (10, 12) and processor (4) in the event that the passive vibration sensor (6) detects that the motion has ceased.

6. A fall detection system (2) as claimed in claim 1, wherein the processor (4) comprises a simple low power processor and a main processor, and wherein the system is configured to provide power to the simple processor in response to the passive vibration sensor (6) detecting motion.

7. A fall detection system (2) as claimed in claim 6, wherein the simple processor is configured to increase a sampling rate of the one or more other sensors (10, 12) in response to the passive vibration sensor (6) detecting motion.

8. A fall detection system (2) as claimed in claim 7, wherein the simple processor is configured to analyze the measurements from the one or more other sensors (10, 12) to identify a characteristic of a fall.

9. A fall detection system (2) as claimed in claim 8, wherein the simple processor is configured to activate the main processor in the event that the simple processor detects a characteristic of a fall.

10. A fall detection system (2) as claimed in claim 7, 8 or 9, wherein the simple processor is configured to reduce the sampling rate of the one or more other sensors (10, 12) in response to the passive vibration sensor (6) detecting that the motion has ceased.

11. A fall detection system (2) as claimed in any preceding claim, wherein the one or more other sensors (10, 12) comprises an accelerometer and/or a barometer.

12. A method of operating a fall detection system for detecting a fall of a user, said fall detection system (2) comprising a passive vibration sensor (6) requiring no electrical energy to operate, one or more other sensors (10, 12) for detecting a movement characteristic of a fall, and a processor (4) for analyzing measurements from the one or more other sensors to determine if a fall has occurred; the method comprising the step of:
selectively providing power to the one or more other sensors and/or processor in response to the passive vibration sensor detecting motion of the user of the fall detection system.

## Patentansprüche

1. Sturzdetektionssystem (2) zum Detektieren eines Sturzes eines Benutzers, das Folgendes umfasst:
einen passiven Vibrationssensor (6), der für seinen Betrieb keine elektrische Energie erfordert;
einen oder mehrere andere Sensoren (10, 12) zum Detektieren einer Bewegungseigenschaft eines Sturzes; und
einen Prozessor (4) zum Analysieren von Messwerten von dem einen oder mehreren anderen Sensoren, um zu ermitteln, ob es zu einem Sturz gekommen ist; wobei das System konfiguriert ist, um den einen oder die mehreren anderen Sensoren und/oder den Prozessor in Reaktion darauf, dass der passive Vibrationssensor eine Bewegung des Benutzers des Sturzdetektionssystems detektiert, selektiv mit Energie zu versorgen.

2. Sturzdetektionssystem (2) nach Anspruch 1, wobei das System weiterhin einen passiven Neigungssensor (8) umfasst; wobei das System konfiguriert ist, um den einen oder mehrere andere Sensoren (10, 12) in Reaktion darauf, dass der passive Vibrationssensor (6) eine Bewegung eines Benutzers erkennt, mit Energie zu versorgen, und um den Prozessor in dem Fall, dass die Messwerte von dem Neigungssensor auf eine Eigenschaft eines Sturzes hinweisen, mit Energie zu versorgen.

3. Sturzdetektionssystem (2) nach Anspruch 1, wobei das System konfiguriert ist, um den einen oder mehrere andere Sensoren (10, 12) in Reaktion darauf, dass der passive Vibrationssensor (6) eine Bewegung eines Benutzers erkennt, mit Energie zu versorgen, und um den Prozessor (4) mit Energie zu versorgen, so dass der Prozessor sich in einem Niederleistungsmodus befindet, um Messwerte von den anderen Sensoren zu analysieren, um Eigenschaften eines Sturzes zu identifizieren.

4. Sturzdetektionssystem (2) nach Anspruch 3, wobei der Prozessor (4) konfiguriert ist, um in dem Fall, dass die Messswerte von einem der anderen Sensoren (10, 12) auf eine Eigenschaft eines Sturzes hinweisen, auf einen Hochleistungsmodus umzuschalten.

5. Sturzdetektionssystem (2) nach einem der vorhergehenden Ansprüche, wobei das System konfiguriert ist, um die dem einen oder mehreren anderen Sensoren (10, 12) und dem Prozessor (4) zugeführte Energie zu stoppen, falls der passive Vibrationssensor (6) detektiert, dass die Bewegung aufgehört hat.

6. Sturzdetektionssystem (2) nach Anspruch 1, wobei der Prozessor (4) einen einfachen Niederleistungsprozessor und einen Hauptprozessor umfasst, und wobei das System konfiguriert ist, um den einfachen Prozessor in Reaktion darauf, dass der passive Vibrationssensor (6) Bewegung erkennt, mit Energie zu versorgen.

7. Sturzdetektionssystem (2) nach Anspruch 6, wobei der einfache Prozessor konfiguriert ist, um eine Abtastrate von dem einen oder mehreren anderen Sensoren (10, 12) in Reaktion darauf, dass der passive Vibrationssensor (6) Bewegung detektiert, zu erhöhen.

8. Sturzdetektionssystem (2) nach Anspruch 7, wobei der einfache Prozessor konfiguriert ist, um die Messwerte von dem einen oder mehreren anderen Sensoren (10, 12) zu analysieren, um eine Eigenschaft eines Sturzes zu identifizieren.

9. Sturzdetektionssystem (2) nach Anspruch 8, wobei der einfache Prozessor konfiguriert ist, um den Hauptprozessor zu aktivieren, falls der einfache Prozessor eine Eigenschaft eines Sturzes detektiert.

10. Sturzdetektionssystem (2) nach Anspruch 7, 8 oder 9, wobei der einfache Prozessor konfiguriert ist, um die Abtastrate von dem einen oder mehreren anderen Sensoren (10, 12) in Reaktion darauf, dass der passive Vibrationssensor (6) detektiert, dass die Bewegung aufgehört hat, zu reduzieren.

11. Sturzdetektionssystem (2) nach einem der vorhergehenden Ansprüche, wobei der eine oder mehrere andere Sensoren (10, 12) einen Beschleunigungsmesser und/oder ein Barometer umfasst.

12. Verfahren zum Betrieb eines Sturzdetektionssystems zum Detektieren eines Sturzes eines Benutzers, wobei das genannte Sturzdetektionssystem (2) Folgendes umfasst: einen passiven Vibrationssensor (6), der für seinen Betrieb keine elektrische Energie erfordert, einen oder mehrere andere Sensoren (10, 12) zum Detektieren einer Bewegungseigenschaft eines Sturzes, und einen Prozessor (4) zum Analysieren von Messwerten von dem einen oder mehreren anderen Sensoren, um zu ermitteln, ob es zu einem Sturz gekommen ist; wobei das Verfahren den folgenden Schritt umfasst:
selektives Versorgen des einen oder der mehreren anderen Sensoren und/oder des Prozessors mit Energie in Reaktion darauf, dass der passive Vibrationssensor eine Bewegung des Benutzers des Sturzdetektionssystems detektiert.

## Revendications

1. Système de détection de chute (2) pour détecter une chute d'un utilisateur, comprenant :
un capteur de vibrations passif (6) dont le fonctionnement ne nécessite pas d'énergie électrique ;
un ou plusieurs autres capteurs (10, 12) pour détecter une caractéristique de mouvement d'une chute ; et
un processeur (4) pour analyser des mesures de l'un ou plusieurs autres capteurs pour déterminer si une chute s'est produite ;
dans lequel le système est configuré pour fournir sélectivement de l'énergie électrique à l'un ou plusieurs autres capteurs et/ou au processeur en réponse à la détection de mouvement de l'utilisateur du système de détection de chute par le capteur de vibrations passif.

2. Système de détection de chute (2) selon la revendication 1, le système comprenant en outre un capteur d'inclinaison passif (8) ; dans lequel le système est configuré pour fournir de l'énergie électrique à l'un ou plusieurs autres capteurs (10, 12) en réponse à la détection d'un mouvement d'un utilisateur par le capteur de vibrations passif (6), et pour fournir de l'énergie électrique au processeur dans le cas où les mesures du capteur d'inclinaison indiquent une caractéristique d'une chute.

3. Système de détection de chute (2) selon la revendication 1, dans lequel le système est configuré pour fournir de l'énergie électrique à l'un ou plusieurs autres capteurs (10, 12) en réponse à la détection d'un mouvement de l'utilisateur par le capteur de vibrations passif (6), et pour fournir de l'énergie électrique au processeur (4) de sorte que le processeur soit en mode de basse puissance pour analyser des mesures des autres capteurs pour identifier des caractéristiques d'une chute.

4. Système de détection de chute (2) selon la revendication 3, dans lequel le processeur (4) est configuré pour passer à un mode de haute puissance dans le cas où les mesures de l'un des autres capteurs (10, 12) indique une caractéristique d'une chute.

5. Système de détection de chute (2) selon l'une quelconque des revendications précédentes, dans lequel le système est configuré pour arrêter de fournir de l'énergie électrique à l'un ou plusieurs autres capteurs (10, 12) et au processeur (4) dans le cas où le capteur de vibrations passif (6) détecte que le mouvement a cessé.

6. Système de détection de chute (2) selon la revendication 1, dans lequel le processeur (4) comprend un processeur de basse puissance simple et un processeur principal, et dans lequel le système est configuré pour fournir de l'énergie électrique au processeur simple en réponse à la détection d'un mouvement par le capteur de vibrations passif (6).

7. Système de détection de chute (2) selon la revendication 6, dans lequel le processeur simple est configuré pour accroître une fréquence d'échantillonnage de l'un ou plusieurs autres capteurs (10, 12) en réponse à la détection d'un mouvement par le capteur de vibrations passif (6).

8. Système de détection de chute (2) selon la revendication 7, dans lequel le processeur simple est configuré pour analyser les mesures de l'un ou plusieurs autres capteurs (10, 12) pour identifier une caractéristique d'une chute.

9. Système de détection de chute (2) selon la revendication 8, dans lequel le processeur simple est configuré pour activer le processeur principal dans le cas où le processeur simple détecte une caractéristique d'une chute.

10. Système de détection de chute (2) selon la revendication 7, 8 ou 9, dans lequel le processeur simple est configuré pour réduire la fréquence d'échantillonnage de l'un ou plusieurs autres capteurs (10, 12) en réponse à la détection par le détecteur de vibrations passif (6) que le mouvement a cessé.

11. Système de détection de chute (2) selon l'une quelconque des revendications précédentes, dans lequel l'un ou plusieurs autres capteurs (10, 12) comprend un accéléromètre et/ou un baromètre.

12. Procédé de fonctionnement d'un système de détection de chute pour détecter une chute d'un utilisateur, ledit système de détection de chute (2) comprenant un capteur de vibrations passif (6) dont le fonctionnement ne nécessite pas d'énergie électrique, un ou plusieurs autres capteurs (10, 12) pour détecter une caractéristique de mouvement d'une chute, et un processeur (4) pour analyser des mesures de l'un ou plusieurs autres capteurs pour déterminer si une chute s'est produite ; le procédé comprenant l'étape consistant à :
fournir sélectivement de l'énergie électrique à l'un ou plusieurs autres capteurs et/ou au processeur en réponse à la détection de mouvement de l'utilisateur du système de détection de chute par le capteur de vibrations passif.
